# EUROPÄISCHE PATENTSCHRIFT

(11) **EP 4 198 012 B1**
(45) Veröffentlichungstag und Bekanntmachung des Hinweises auf die Patenterteilung: **15.05.2024**
(21) Anmeldenummer: 21215374.6
(22) Anmeldetag: 17.12.2021
(51) Int. Cl.: C07C 45/50, C07C 47/02

(54) **VERFAHREN ZUR HYDROFORMYLIERUNG VON OLEFINEN UNTER EINSATZ VON PT UND IOD ODER BROM**
METHOD FOR THE HYDROFORMYLATION OF OLEFINS USING PT AND IODINE OR BROMINE
PROCÉDÉ D'HYDROFORMYLATION D'OLÉFINES À L'AIDE DU PT ET DE L'IODE OU DU BROME

(43) Veröffentlichungstag der Anmeldung: 21.06.2023
(73) Patentinhaber: Evonik Oxeno GmbH & Co. KG, 45772 Marl (DE)
(72) Erfinder: SCHNEIDER, Carolin, 40789 Monheim am Rhein (DE); JACKSTELL, Ralf, 18106 Rostock (DE); BELLER, Matthias, 18211 Ostseebad Nienhagen (DE); FRANKE, Robert, 45772 Marl (DE)
(74) Vertreter: Evonik Patent Association

(56) Entgegenhaltungen:
- VEEN VAN DER L A ET AL: "WIDE BITE ANGLE AMINE, ARSINE AND PHOSPHINE LIGANDS IN RHODIUM-AND PLATINUM/TIN-CATALYSED HYDROFORMYLATION", JOURNAL OF THE CHEMICAL SOCIETY, DALTON TRANSACTIONS, CHEMICAL SOCIETY. LETCHWORTH, GB, Bd. 13, 1. Januar 2000 (2000-01-01), Seiten 2105-2112, XP001084858, ISSN: 1472-7773, DOI: 10.1039/B002126L

## Beschreibung

Die vorliegende Erfindung betrifft ein Verfahren zur Hydroformylierung von Olefinen unter Einsatz von Pt und lod oder Brom.

In L. A. van der Veen et al., Journal of the Chemical Society, Dalton Transactions, Bd. 13, 2000, Seiten 2105-2112 (DOI: 10.1039/B002126L) wird der Einsatz Amin-, Arsin- und Phosphin-Liganden in der Rhodium- und Platin/Zinn-katalysierten Hydroformylierung beschrieben.

In C. Botteghi et al., Journal of Molecular Catalysis A: Chemical 200, (2003), 147-156 wird der Einsatz von Pt(Xantphos)Cl₂ zur Hydroformylierung von 2-Tosyloxystyrol beschrieben.

Der vorliegende Erfindung lag die Aufgabe zugrunde, eine neues Hydroformylierungsverfahren bereitzustellen. Das Verfahren soll hierbei eine gegenüber dem aus dem Stand der Technik unter Einsatz von Pt mit Cl₂ bekannten Verfahren gesteigerte Ausbeute liefern.

Diese Aufgabe wird gelöst durch ein Verfahren gemäß Anspruch 1.

Verfahren umfassend die Verfahrensschritte:
a) Vorlegen eines Olefins;
b) Zugabe einer Verbindung gemäß der Formel (I): 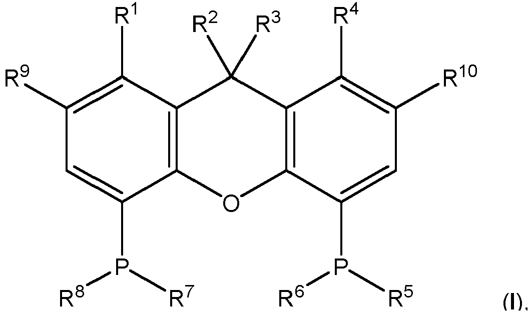
   wobei R¹, R², R³, R⁴, R⁵, R⁶, R⁷, R⁸ ausgewählt sind aus: -H, -(C₁-C₁₂)-Alkyl, -(C₆-C₂₀)-Aryl, und R⁹, R¹⁰ ausgewählt sind aus: -(C₁-C₁₂)-Alkyl, -(C₆-C₂₀)-Aryl,
   und für den Fall, dass R¹, R², R³, R⁴, R⁵, R⁶, R⁷, R⁸, R⁹, R¹⁰ für -(C₆-C₂₀)-Aryl stehen, kann der Arylring Substituenten aufweisen, welche ausgewählt sind aus: -(C₁-C₁₂)-Alkyl, -O-(C₁-C₁₂)-Alkyl;
c) Zugabe einer Pt-Verbindung, welche zur Ausbildung eines Komplexes befähigt ist;
d) Zugabe einer lod-Verbindung oder Brom-Verbindung;
e) Zuführen von CO und H₂;
f) Erwärmen des Reaktionsgemisches aus a) bis e), wobei das Olefin zu einem Aldehyd umgesetzt wird.

Hierbei können die Verfahrensschritte a) bis e) in beliebiger Reihenfolge erfolgen. Üblicherweise erfolgt die Zugabe von CO und H₂ jedoch, nachdem die Reaktionspartner in den Schritten a) bis d) vorgelegt wurden.

Hierbei können die Verfahrensschritte c) und d) in einem Schritt, durch Zugabe von PtI₂ oder PtBr₂, erfolgen.

In einer bevorzugten Variante des Verfahrens erfolgt die Zugabe der Pt-Verbindung und der lod-Verbindung oder Brom-Verbindung in einem Schritt, durch Zugabe von PtI₂ oder PtBr₂.

Der Begriff (C₁-C₁₂)-Alkyl umfasst geradkettige und verzweigte Alkylgruppen mit 1 bis 12 Kohlenstoffatomen. Vorzugsweise handelt es sich dabei um (C₁-C₈)-Alkylgruppen, besonders bevorzugt (C₁-C₆)-Alkyl, am meisten bevorzugt (C₁-C₄)-Alkyl.

Geeignete (C₁-C₁₂)-Alkylgruppen sind insbesondere Methyl, Ethyl, Propyl, Isopropyl, *n*-Butyl, *iso*-Butyl, *sec*-Butyl, *tert*-Butyl, *n*-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,1-Dimethylbutyl, 1,2-Dimethylbutyl, 2,2-Dimethylbutyl, 1,3-Dimethylbutyl, 2,3-Dimethylbutyl, 3,3-Dimethylbutyl, 1,1,2-Trimethylpropyl, 1,2,2-Trimethylpropyl, 1-Ethylbutyl, 1-Ethyl-2-methylpropyl, *n*-Heptyl, 2-Heptyl, 3-Heptyl, 2-Ethylpentyl, 1-Propylbutyl, n-Octyl, 2-Ethylhexyl, 2-Propylheptyl, Nonyl, Decyl.

Der Begriff (C₆-C₂₀)-Aryl umfasst mono- oder polycyclische aromatische Kohlenwasserstoffreste mit 6 bis 20 Kohlenstoffatomen. Vorzugsweise handelt es sich dabei um (C₆-C₁₄)-Aryl, besonders bevorzugt (C₆-C₁₀)-Aryl.

Geeignete (C₆-C₂₀)-Arylgruppen sind insbesondere Phenyl, Naphthyl, Indenyl, Fluorenyl, Anthracenyl, Phenanthrenyl, Naphthacenyl, Chrysenyl, Pyrenyl, Coronenyl. Bevorzugte (C₆-C₂₀)-Arylgruppen sind Phenyl, Naphthyl und Antracenyl.

In einer Variante des Verfahrens sind R², R³, R⁵, R⁶, R⁷, R⁸ ausgewählt aus: -(C₁-C₁₂)-Alkyl, - (C₆-C₂₀)-Aryl.

In einer Variante des Verfahrens stehen R⁵, R⁶, R⁷, R⁸ für -(C₆-C₂₀)-Aryl.

In einer Variante des Verfahrens stehen R⁵ und R⁶ für unterschiedliche Reste und R⁷ und R⁸ für unterschiedliche Reste.

In einer Variante des Verfahrens stehen R² und R³ für -(C₁-C₁₂)-Alkyl.

In einer Variante des Verfahrens stehen R² und R⁸ für -CH₃.

In einer Variante des Verfahrens stehen R¹ und R⁴ für -H.

In einer Variante des Verfahrens stehen R⁹ und R¹⁰ für -(C₁-C₁₂)-Alkyl.

In einer Variante des Verfahrens stehen R⁹ und R¹⁰ für -^{t}Bu.

In einer Variante des Verfahrens weisen die Verbindung (I) die Struktur (1) auf:

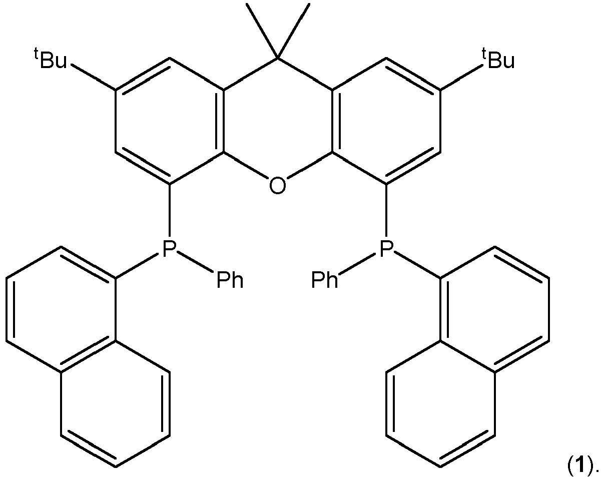

In einer Variante des Verfahrens ist die Pt-Verbindung ausgewählt aus: Pt(II)I₂, Pt(II)Br₂, Pt(IV)I₄, Pt(IV)Br₄, Diphenyl(1,5-COD)Pt(II), Pt(II)(acac)₂, Pt(0)(PPh₃)₄, Pt(0)(DVTS)-Lösung (CAS: 68478-92-2), Pt(0)(Ethylen)(PPh₃)₂, Pt(II)Br₂(COD), Tris(benzylidenaceton)Pt(0), Pt(II)(OAC)₂ Lösung, Pt(0)(t-Bu)₂, Pt(II)(COD)Me₂, Pt(II)(COD)I₂, Pt(IV)IMe₃, Pt(II)(Hexafluoroacetylacetonat)₂.

In einer Variante des Verfahrens ist die Pt-Verbindung ausgewählt aus: Pt(II)I₂, Pt(II)Br₂.

In einer Variante des Verfahrens ist die lod-Verbindung beziehungsweise die Brom-Verbindung ausgewählt aus: Alkalihalogenid, Erdalkalihalogenid, NH₄X, Alkylammoniumhalogenid, Dialkylhalogenid, Trialkylhalogenid, Tetraalkylhalogenid, Cycloalkylammoniumhalogenid.

In einer Variante des Verfahrens wird in Verfahrensschritt d) eine lod-Verbindung zugegeben.

In einer Variante des Verfahrens ist die lod-Verbindung Pt(II)I₂.

In einer Variante des Verfahrens wird die lod-Verbindung in einer Menge zugegeben, gemessen in Äquivalenten bezogen auf Pt, welche im Bereich von 0.1 bis 10 liegt.

In einer Variante des Verfahrens wird in Verfahrensschritt d) eine Brom-Verbindung zugegeben.

In einer Variante des Verfahrens ist die Brom-Verbindung Pt(II)Br₂.

In einer Variante des Verfahrens wird die Brom-Verbindung in einer Menge zugegeben, gemessen in Äquivalenten bezogen auf Pt, welche im Bereich von 0.1 bis 10 liegt.

In einer Variante des Verfahrens umfasst dieses den zusätzlichen Verfahrensschritt e'): e') Zugabe eines Lösungsmittels.

In einer Variante des Verfahrens ist das Lösungsmittel ausgewählt aus: THF, DCM, ACN, Heptan, DMF, Toluol, Texanol, Pentan, Hexan, Octan, Isooctan, Decan, Dodecan, Cyclohexan, Benzen, Xylene, Marlotherm, Propylencarbonat, MTBE, Diglyme, Triglyme, Diethylether, Dioxan, Isopropanol, Tertbutanol, Isononanol, Isobutanol, Isopentanol, Ethylacetat.

In einer Variante des Verfahrens ist das Lösungsmittel ausgewählt aus: THF, DCM, ACN, Heptan, DMF, Toluol, Texanol.

In einer Variante des Verfahrens erfolgt das Zuführen von CO und H₂ bei einem Druck in einem Bereich von 1 MPa (10 bar) bis 6 MPa (60 bar).

In einer Variante des Verfahrens erfolgt das Zuführen von CO und H₂ bei einem Druck in einem Bereich von 1 MPa (20 bar) bis 6 MPa (50 bar).

In einer Variante des Verfahrens erfolgt das Erwärmen auf eine Temperatur im Bereich von 25 °C bis 150°C.

In einer Variante des Verfahrens erfolgt das Erwärmen auf eine Temperatur im Bereich von 30 °C bis 130°C.

In einer Variante des Verfahrens ist das Olefin ausgewählt aus: Ethen, Propen, 1-Buten, cis- und/oder trans-2-Buten, iso-Buten, 1,3-Butadien, 1-Penten, cis- und/oder trans-2-Penten, 2-Methyl-1-buten, 3-Methyl-1-buten, 2-Methyl-2-buten, Hexen, Tetramethylethylen, Hepten, 1-Octen, 2-Octen, Di-n-Buten, oder Mischungen davon.

Im Folgenden soll die Erfindung anhand von Ausführungsbeispielen näher erläutert werden.

### Versuchsbeschreibung

Ein Vial wurde mit PtX₂ (X = Halogen), Ligand, und einem im Ofen getrockneten Rührstab bestückt. Dann wird das Vial mit Septum (PTFE-beschichteter StyrolButadien-Kautschuk) und Phenolharzdeckel verschlossen. Das Vial wird dreimal evakuiert und mit Argon wieder befüllt. Mit einer Spritze wurden Toluol und 1-Octen in das Vial gegeben. Das Vial wurde in eine Legierungsplatte gestellt, die in einen Autoklav der Reihe 4560 von Parr Instruments unter Argon Atmosphäre überführt wurde. Nach dreimaligem Spülen des Autoklaven mit CO/H₂ wurde der Synthesegasdruck auf 40 bar bei Raumtemperatur erhöht. Die Reaktion wurde 18 h bei 80 °C durchgeführt. Nach Beendigung der Reaktion wurde der Autoklav auf Raumtemperatur abgekühlt und vorsichtig entspannt. Der Ausbeute und Selektivität wurden durch GC-Analyse bestimmt.

### Variation des Halogens

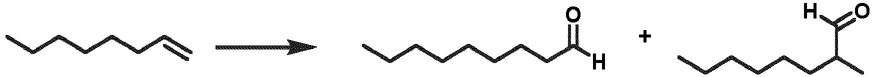

### Reaktionsbedingungen:

1.0 mmol 1-Octen, 0.5 mol% PtX₂, 2,0 Äquivalente Ligand (1), Lösungsmittel: Toluol, p(CO/H₂): 40 bar, T: 80 °C, t: 18 h.

### Ausbeuten:

| Ligand | Halogen | Ausbeute [%] |
|---|---|---|
| 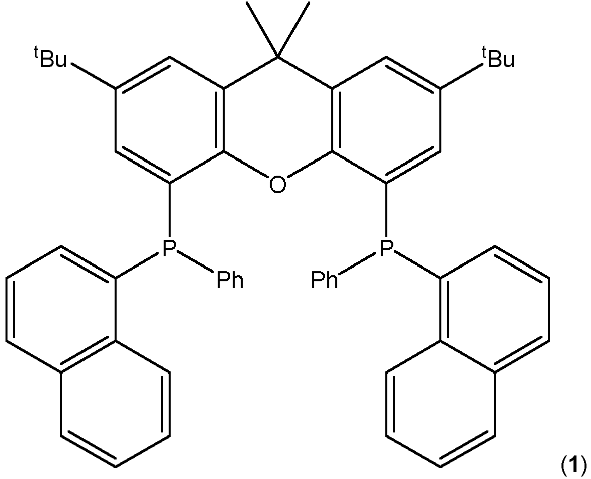 | I / Br / Cl | 96 / 24 / 0 |

Wie die Versuchsergebnisse zeigen, wird die Aufgabe durch das erfindungsgemäße Verfahren gelöst. Der Versuch mit X = Cl ist ein Vergleichsversuch.

## Patentansprüche

1. Verfahren umfassend die Verfahrensschritte:
a) Vorlegen eines Olefins;
b) Zugabe einer Verbindung gemäß der Formel (I): 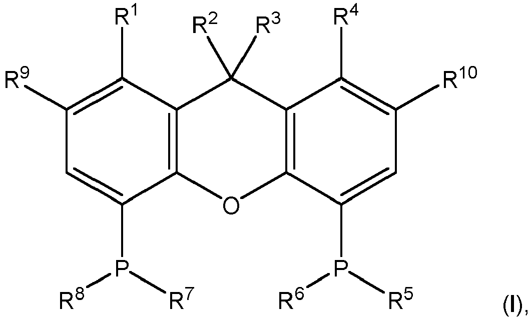
wobei R¹, R², R³, R⁴, R⁵, R⁶, R⁷, R⁸ ausgewählt sind aus: -H, -(C₁-C₁₂)-Alkyl, -(C₆-C₂₀)-Aryl, und R⁹, R¹⁰ ausgewählt sind aus: -(C₁-C₁₂)-Alkyl, -(C₆-C₂₀)-Aryl,
und für den Fall, dass R¹, R², R³, R⁴, R⁵, R⁶, R⁷, R⁸, R⁹, R¹⁰ für -(C₆-C₂₀)-Aryl stehen, kann der Arylring Substituenten aufweisen, welche ausgewählt sind aus: -(C₁-C₁₂)-Alkyl, -O-(C₁-C₁₂)-Alkyl;
c) Zugabe einer Pt-Verbindung, welche zur Ausbildung eines Komplexes befähigt ist;
d) Zugabe einer lod-Verbindung oder Brom-Verbindung;
e) Zuführen von CO und H₂;
f) Erwärmen des Reaktionsgemisches aus a) bis e), wobei das Olefin zu einem Aldehyd umgesetzt wird.

2. Verfahren nach Anspruch 1,
wobei R², R³, R⁵, R⁶, R⁷, R⁸ ausgewählt sind aus: -(C₁-C₁₂)-Alkyl, -(C₆-C₂₀)-Aryl.

3. Verfahren nach einem der Ansprüche 1 oder 2,
wobei R⁵, R⁶, R⁷, R⁸ für -(C₆-C₂₀)-Aryl stehen.

4. Verfahren nach einem der Ansprüche 1 bis 3,
wobei R² und R³ für -(C₁-C₁₂)-Alkyl stehen.

5. Verfahren nach einem der Ansprüche 1 bis 4,
wobei R¹ und R⁴ für -H stehen.

6. Verfahren nach einem der Ansprüche 1 bis 5,
wobei R⁹ und R¹⁰ für -(C₁-C₁₂)-Alkyl stehen.

7. Verfahren nach einem der Ansprüche 1 bis 6, wobei die Verbindung (I) die Struktur (1) aufweist:

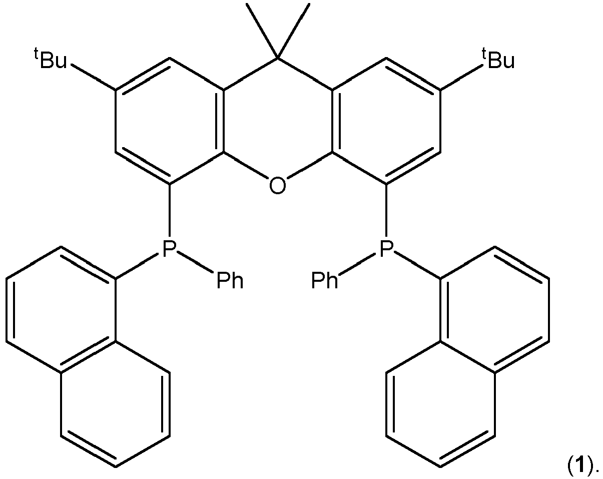

8. Verfahren nach einem der Ansprüche 1 bis 7,
wobei die Pt-Verbindung ausgewählt ist aus: Pt(II)I₂, Pt(II)Br₂, Pt(IV)I₄, Pt(IV)Br₄, Diphenyl(1,5-COD)Pt(II), Pt(II)(acac)₂, Pt(0)(PPh₃)₄, Pt(0)(DVTS)-Lösung (CAS: 68478-92-2), Pt(0)(Ethylen)(PPh₃)₂, Pt(II)Br₂(COD), Tris(benzylidenaceton)Pt(0), Pt(II)(OAC)₂ Lösung, Pt(0)(t-Bu)₂, Pt(II)(COD)Me₂, Pt(II)(COD)I₂, Pt(IV)IMe₃, Pt(II)(Hexafluoroacetylacetonat)₂.

9. Verfahren nach einem der Ansprüche 1 bis 8,
wobei in Verfahrensschritt d) eine lod-Verbindung zugegeben wird.

10. Verfahren nach Anspruch 9,
wobei die lod-Verbindung in einer Menge zugegeben wird, gemessen in Äquivalenten bezogen auf Pt, welche im Bereich von 0.1 bis 10 liegt.

11. Verfahren nach einem der Ansprüche 1 bis 8,
wobei in Verfahrensschritt d) eine Brom-Verbindung zugegeben wird.

12. Verfahren nach Anspruch 11,
wobei die Brom-Verbindung in einer Menge zugegeben wird, gemessen in Äquivalenten bezogen auf Pt, welche im Bereich von 0.1 bis 10 liegt.

13. Verfahren nach einem der Ansprüche 1 bis 12,
umfassend den zusätzlichen Verfahrensschritt e'):
e') Zugabe eines Lösungsmittels.

14. Verfahren nach einem der Ansprüche 1 bis 13,
wobei das Zuführen von CO und H₂ bei einem Druck erfolgt in einem Bereich von 1 MPa (10 bar) bis 6 MPa (60 bar).

15. Verfahren nach einem der Ansprüche 1 bis 14,
wobei das Erwärmen auf eine Temperatur erfolgt im Bereich von 25 °C bis 150°C.

## Claims

1. Process comprising the process steps of:
a) initially charging an olefin;
b) adding a compound of formula **(I):** 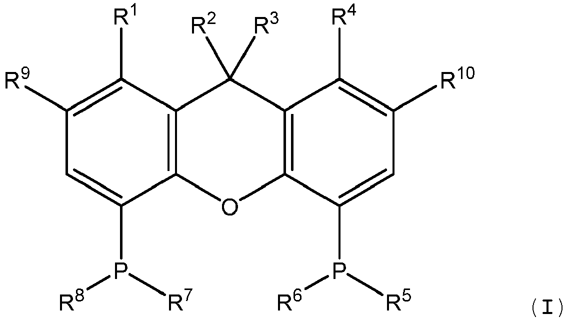
where R¹, R², R³, R⁴, R⁵, R⁶, R⁷, R⁸ are selected from: -H, - (C₁-C₁₂) -alkyl, - (C₆-C₂₀) -aryl, and
R⁹, R¹⁰ are selected from: - (C₁-C₁₂) -alkyl, - (C₆-C₂₀)-aryl, and, if R¹, R², R³, R⁴, R⁵, R⁶, R⁷, R⁸, R⁹, R¹⁰ are -(C₆-C₂₀)-aryl, the aryl ring may have substituents selected from: - (C₁-C₁₂) -alkyl, -O-(C₁-C₁₂) -alkyl;
c) adding a Pt compound capable of forming a complex;
d) adding an iodine compound or bromine compound;
e) feeding in CO and H₂;
f) heating the reaction mixture from steps a) to e), to convert the olefin to an aldehyde.

2. Process according to Claim 1,
where R², R³, R⁵, R⁶, R⁷, R⁸ are selected from: -(C₁-C₁₂)-alkyl, -(C₆-C₂₀)-aryl.

3. Process according to either of Claims 1 and 2,
where R⁵, R⁶, R⁷, R⁸ are -(C₆-C₂₀)-aryl.

4. Process according to any of Claims 1 to 3,
where R² and R³ are - (C₁-C₁₂)-alkyl.

5. Process according to any of Claims 1 to 4,
where R¹ and R⁴ are each -H.

6. Process according to any of Claims 1 to 5,
where R⁹ and R¹⁰ are -(C₁-C₁₂)-alkyl.

7. Process according to any of Claims 1 to 6,
wherein the compound **(I)** has the structure **(1):**

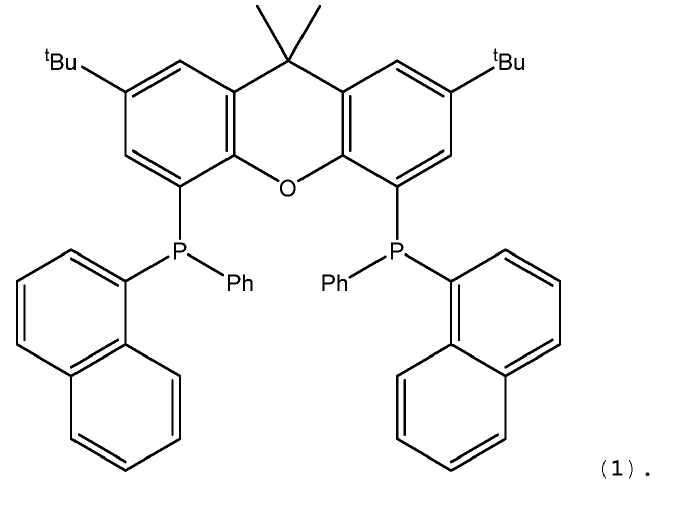

8. Process according to any of Claims 1 to 7,
wherein the Pt compound is selected from: Pt(II)I₂, Pt(II)Br₂, Pt(IV)I₄, Pt(IV)Br₄, diphenyl (1,5-COD)Pt(II), Pt(II)(acac)₂, Pt(0)(PPh₃)₄, Pt(0) (DVTS) solution (CAS: 68478-92-2), Pt(0) (ethylene) (PPh₃)₂, Pt(II)Br₂(COD), tris(benzylideneacetone)Pt(0), Pt(II)(OAC)₂ solution, Pt(0)(t-Bu)₂, Pt(II)(COD)Me₂, Pt(II) (COD)I₂, Pt(IV)IMe₃, Pt(II)(hexafluoroacetylacetonate)₂.

9. Process according to any of Claims 1 to 8,
wherein an iodine compound is added in process step d).

10. Process according to Claim 9,
wherein the iodine compound is added in an amount in the range of 0.1 to 10, measured in equivalents based on Pt.

11. Process according to any of Claims 1 to 8,
wherein a bromine compound is added in process step d).

12. Process according to Claim 11,
wherein the bromine compound is added in an amount in the range of 0.1 to 10, measured in equivalents based on Pt.

13. Process according to any of Claims 1 to 12, comprising the additional process step e'): e') adding a solvent.

14. Process according to any of Claims 1 to 13,
wherein CO and H₂ is fed in at a pressure in a range from 1 MPa (10 bar) to 6 MPa (60 bar).

15. Process according to any of Claims 1 to 14,
wherein the reaction mixture is heated to a temperature in the range from 25°C to 150°C.

## Revendications

1. Procédé comprenant les étapes de procédé :
a) disposition préalable d'une oléfine ;
b) ajout d'un composé selon la formule (I) : 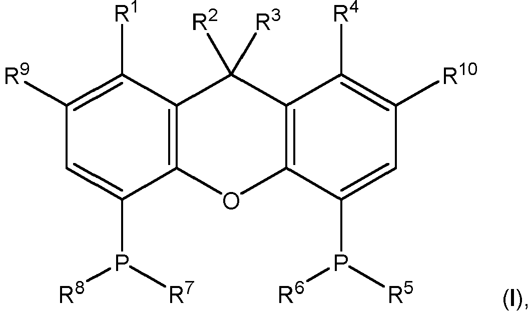
dans laquelle R¹, R², R³, R⁴, R⁵, R⁶, R⁷, R⁸ sont choisis parmi : -H, - (C₁-C₁₂) -alkyle, - (C₆-C₂₀) -aryle et
R⁹, R¹⁰ sont choisis parmi : - (C₁-C₁₂) -alkyle, - (C₆-C₂₀) - aryle,
et pour le cas où R¹, R², R³, R⁴, R⁵, R⁶, R⁷, R⁸, R⁹, R¹⁰ représentent -(C₆-C₂₀)-aryle, le cycle aryle peut présenter des substituants qui sont choisis parmi : -(C₁-C₁₂)-alkyle, -O-(C₁-C₁₂)-alkyle ;
c) ajout d'un composé de Pt, qui est apte à la formation d'un complexe ;
d) ajout d'un composé d'iode ou d'un composé de brome ;
e) introduction de CO et de H₂ ;
f) chauffage du mélange réactionnel de a) à e), l'oléfine étant transformée en aldéhyde.

2. Procédé selon la revendication 1, R², R³, R⁵, R⁶, R⁷, R⁸ étant choisis parmi : -(C₁-C₁₂)-alkyle, - (C₆-C₂₀) -aryle.

3. Procédé selon l'une des revendications 1 ou 2, R⁵, R⁶, R⁷, R⁸ représentant -(C₆-C₂₀) -aryle.

4. Procédé selon l'une des revendications 1 à 3, R² et R³ représentant -(C₁-C₁₂) -alkyle.

5. Procédé selon l'une des revendications 1 à 4, R¹ et R⁴ représentant -H.

6. Procédé selon l'une des revendications 1 à 5, R⁹ et R¹⁰ représentant -(C₁-C₁₂) -alkyle.

7. Procédé selon l'une des revendications 1 à 6, le composé (I) présentant la structure (1) :

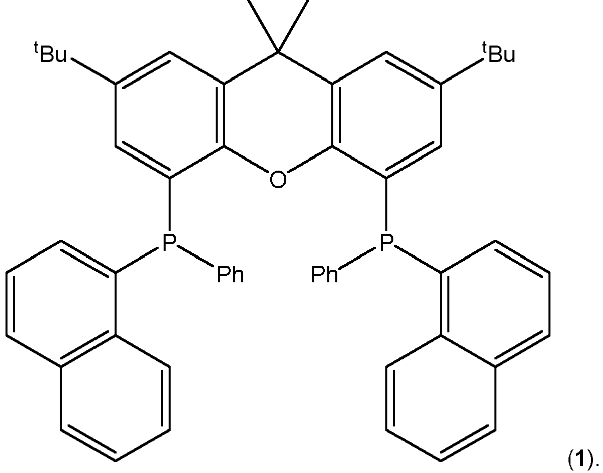

8. Procédé selon l'une des revendications 1 à 7, le composé de Pt étant choisi parmi : Pt(II)I₂, Pt(II)Br₂, Pt(IV)I₄, Pt(IV)Br₄, diphényl(1,5-COD)Pt(II), Pt(II) (acac)₂, Pt(0)(PPh₃)₄, solution de Pt(0)(DVTS) (CAS : 68478-92-2), Pt(0)(éthylène) (PPh₃)₂, Pt(II)Br₂(COD), tris(benzylidénacétone)Pt(0), solution de Pt(II)(OAC)₂, Pt(0)(t-Bu)₂, Pt(II)(COD)Me₂, Pt(II)(COD)I₂, Pt(IV)IMe₃, Pt(II)(hexafluoroacétylacétonate)₂.

9. Procédé selon l'une des revendications 1 à 8, un composé d'iode étant ajouté dans l'étape de procédé d).

10. Procédé selon la revendication 9, le composé d'iode étant ajouté en une quantité, mesurée en équivalents par rapport au Pt, qui est située dans la plage de 0,1 à 10.

11. Procédé selon l'une des revendications 1 à 8, un composé de brome étant ajouté dans l'étape de procédé d).

12. Procédé selon la revendication 11, le composé de brome étant ajouté en une quantité, mesurée en équivalents par rapport au Pt, qui est située dans la plage de 0,1 à 10.

13. Procédé selon l'une des revendications 1 à 12, comprenant l'étape de procédé supplémentaire e') : e') ajout d'un solvant.

14. Procédé selon l'une des revendications 1 à 13, l'introduction de CO et de H₂ ayant lieu à une pression dans une plage de 1 MPa (10 bars) à 6 MPa (60 bars).

15. Procédé selon l'une des revendications 1 à 14, le chauffage ayant lieu à une température dans la plage de 25°C à 150°C.
